# EUROPÄISCHE PATENTANMELDUNG

(11) **EP 2 623 088 A2**
(43) Veröffentlichungstag der Anmeldung: **07.08.2013**
(21) Anmeldenummer: 12193191.9
(22) Anmeldetag: 19.11.2012
(51) Int. Cl.: A61K 8/31, A61K 8/37, A61Q 5/00, A61K 8/58, A61Q 5/12, A61K 8/81, A61K 8/92

(54) **Haarpflegemittel**

(30) Priorität: 08.12.2011 DE 102011087972; 08.12.2011 DE 102011087973
(71) Anmelder: Henkel AG&Co. KGAA, 40589 Düsseldorf (DE)
(72) Erfinder: Battermann, Marlene, 21271 Asendorf (DE); Hippe, Thomas, 25482 Appen (DE)

(57) **Zusammenfassung**

Kosmetisches Mittel, enthaltend in einem kosmetisch akzeptablen Träger
a) mindestens ein Esteröl
b) mindestens ein Isoparaffin aus der Gruppe Isodecan, Isoundecan, Isododecan, Isotridecan und Isotetradecan

eignen sich in besonderer Weise zur Pflege keratinischer Fasern.

## Beschreibung

Die Anmeldung beschreibt kosmetische Mittel auf der Grundlage einer Wirkstoffkombination aus spezifischen Isoparaffinen und Esterölen sowie die Verwendung dieser Mittel zur Pflege keratinischer Fasern.

Das menschliche Haar ist neben den natürlichen Umwelteinflüssen einer Reihe weiterer, insbesondere kosmetischer Beanspruchungen ausgesetzt. Zu diesen, das Haar strapazierenden Beanspruchungen zählen beispielsweise die Färbung des Haars sowie dessen Verformung, beispielsweise durch eine Dauerwelle. Zur Minderung der nachteiligen Auswirkungen der die Haarstruktur beeinträchtigenden (Umwelt)Einflüsse aber auch zur Erhaltung und Verbesserung der natürlichen Haarstruktur werden kosmetische Haarpflegemittel eingesetzt. Ein wesentlicher Wirkstoff in vielen dieser kosmetischen Mittel sind die Silicium-organischen Verbindungen, insbesondere die Silikone wie Trisiloxane, die sich durch pflegende Eigenschaften auszeichnen. Die Nachteile dieser Silikone sind die durch Benetzung der Haaroberfläche verminderte Penetration von Wirk- und Hilfsstoffen in das Haar und die ebenfalls durch die Benetzung der Haaroberfläche bedingte Erschwerung der Frisurgestaltung. Die Bereitstellung Silikon armer oder Silikon freier Pflegemittel ist daher eine relevante Aufgabe im Bereich der Haarkosmetik.

Als eine von zahlreichen zur Substitution der Silikone geeignete Wirkstoffklasse werden im Stand der Technik die Isoparaffine diskutiert. So beschreiben beispielsweise die europäischen Patente EP 413 417 B1 (Colgate Palmolive) und EP 1 284 712 B1 (Procter&Gamble) pflegende Haarshampoos bzw. Haarkonditionierer, die neben weiteren Bestandteilen auch Isoparaffine enthalten können. Diese Haarpflegemittel weisen jedoch weiterhin eine Reihe von Nachteilen auf und trotz des bisher Erreichten besteht weiterhin ein Bedarf an Trisiloxan armen oder Trisiloxan freien Haarpflegemitteln auf Isoparaffinbasis.

Der Einsatz von Esterölen in haarkonditionierenden Mitteln wird in der internationalen Patentanmeldung WO 2010/063565 A1 (Henkel) beschrieben.

Es wurde nun festgestellt, dass durch die Kombination spezifischer Isoparaffine mit Esterölen Haarpflegemittel mit ausgezeichneter Pflegewirkung erhalten werden können. Diese Mittel bewirken gute Glanzeigenschaften, ein verbessertes Aussehen und eine verbesserte Regeneration der keratinischen Fasern.

Ein erster Gegenstand dieser Anmeldung ist daher ein kosmetisches Mittel, enthaltend in einem kosmetisch akzeptablen Träger
a) mindestens ein Esteröl und
b) mindestens ein Isoparaffin aus der Gruppe Isodecan, Isoundecan, Isododecan, Isotridecan und Isotetradecan.

Es wurde weiter festgestellt, dass durch die Kombination von polymeren quartären Ammoniumverbindungen mit Esterölen Haarpflegemittel mit ausgezeichneter Pflegewirkung erhalten werden können. Diese Mittel bewirken gute Glanzeigenschaften, ein verbessertes Aussehen und eine verbesserte Regeneration der keratinischen Fasern. Besonders bevorzugt können die polymeren quarternären Ammoniumverbindungen zu der erfindungsgemäßen Kombination aus mindestens einem Isoparaffin und mindestens einem Esteröl hinzugefügt werden.

Ein zweiter Gegenstand dieser Anmeldung ist daher ein kosmetisches Mittel, enthaltend in einem kosmetisch akzeptablen Träger
a) mindestens ein Esteröl und
b) mindestens eine polymere quartäre Ammoniumverbindung.

Ein dritter Gegenstand dieser Anmeldung ist weiterhin ein kosmetisches Mittel, enthaltend in einem kosmetisch akzeptablen Träger
a) mindestens ein Esteröl,
b) mindestens ein Isoparaffin aus der Gruppe Isodecan, Isoundecan, Isododecan, Isotridecan und Isotetradecan und
c) mindestens eine polymere quartäre Ammoniumverbindung.

Die erfindungsgemäßen kosmetischen Mittel enthalten als ersten wesentlichen Bestandteil mindestens ein Esteröl. Unter Esterölen werden Ester von C₆ - C₃₀ - Fettsäuren mit C₂ - C₃₀ - Fettalkoholen verstanden. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen.

Erfindungsgemäß besonders bevorzugt sind 2-Ethylhexylpalmitat (Cegesoft^{®} 24), Isopropylmyristat (Rilanit^{®} IPM), Isononansäure-C16-18-alkylester (Cetiol^{®} SN), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{o} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat

(Cetiol^{®} MM), Cetearyl Isononanoate (Cetiol^{®} SN), Ölsäuredecylester (Cetiol^{®} V). Bevorzugte kosmetische Mittel sind dadurch gekennzeichnet, dass als Esteröl 2-Ethylhexylpalmitat eingesetzt wird.

Durch die Kombination von 2-Ethylhexylpalmitat mit den Isoparaffinen kann in den erfindungsgemäßen Mitteln bei gleich bleibender Pflegeleistung insbesondere der Gehalt an Cyclomethiconen abgesenkt werden. Bevorzugte erfindungsgemäße kosmetische Mittel sind daher dadurch gekennzeichnet, dass sie bezogen auf ihr Gesamtgewicht, weniger als 5,0 Gew.-%, vorzugsweise weniger als 2,0 Gew.-%, bevorzugt weniger als 0,5 Gew.-% und insbesondere kein Cyclomethicon enthalten.

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein. Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt. 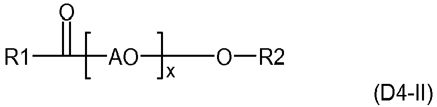
R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Ein erfindungsgemäß besonders bevorzugtes Esteröl ist beispielsweise unter der INCI - Bezeichnung PPG-3 Benzyl Ether Myristate, beispielsweise dem Verkaufsprodukt Crodamol^{®} STS erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 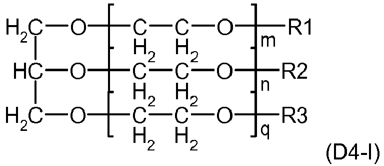
   in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Selbstverständlich ist es erfindungsgemäß auch möglich mehr als zwei Esteröle gleichzeitig zu verwenden. In jedem Falle werden die Esteröle so ausgewählt, dass die Esteröle entsprechend ihren Spreitwerten miteinander kombiniert werden. Die Kombination von 2-Ethylhexylpalmitat und Isopropylmyristat hat sich in den erfindungsgemäßen Mitteln als kosmetisch besonders vorteilhaft erwiesen. Das Gewichtsverhältnis von 2-Ethylhexylpalmitat zu Isopropylmyristat beträgt vorzugsweise 10:1 bis 1:3, bevorzugt 8:1 bis 1:2 und insbesondere 6.1 bis 1:1.

Der Gewichtsanteil des Esteröls am Gesamtgewicht des kosmetischen Mittels beträgt vorzugsweise 10 bis 90 Gew.-%, bevorzugt 20 bis 80 Gew.-% und insbesondere 40 bis 60 Gew.-%.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Esteröl | 10 bis 90 | 20 bis 80 | 40 bis 60 | 20 | 50 |
| Isoparaffin *, Misc | add 100 | add 100 | add 100 | add 100 | add 100 |

| | Formel 6 | Formel 7 | Formel 8 | Formel 9 | Formel 10 |
|---|---|---|---|---|---|
| 2-Ethylhexylpalmitat | 10 bis 90 | 20 bis 80 | 40 bis 60 | 20 | 50 |
| Isoparaffin *, Misc | add 100 | add 100 | add 100 | add 100 | add 100 |

| | Formel 11 | Formel 12 | Formel 13 | Formel 14 | Formel 15 |
|---|---|---|---|---|---|
| 2-Ethylhexylpalmitat | 10 bis 90 | 20 bis 80 | 40 bis 60 | 20 | 40 |
| Isopropylmyristat | 2 bis 35 | 5 bis 30 | 10 bis 25 | 20 | 20 |
| Isoparaffin *, Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isodecan, Isoundecan, Isododecan, Isotridecan und Isotetradecan. | | | | | |

Die erfindungsgemäßen kosmetischen Mittel enthalten als zweiten wesentlichen Bestandteil mindestens ein Isoparaffin aus der Gruppe Isodecan, Isoundecan, Isododecan, Isotridecan und Isotetradecan. Diese Isoparaffine zeichnen sich gegenüber den n-Paraffinen mit gleicher Anzahl an Kohlenstoffatomen bei Einsatz in den kosmetischen Mitteln durch verbesserte olfaktorische Eigenschaften aus. Gegenüber den n-Paraffinen mit gleicher Anzahl an Kohlenstoffatomen sowie gegenüber den Isoparaffinen mit geringerer oder höherer Anzahl an Kohlenstoffatomen wird in Kombination mit den Esterölen zudem überraschend eine verbesserte kosmetische, insbesondere haarpflegende Wirkung erreicht.

Als für die kosmetische Wirkung besonders vorteilhaft hat sich der Einsatz von Isoparaffinen aus der Gruppe Isoundecan, Isododecan und Isotridecan erwiesen. Entsprechende kosmetische Mittel, dadurch gekennzeichnet, dass sie mindestens ein Isoparaffin ausgewählt aus der Gruppe Isoundecan, Isododecan, Isotridecan enthalten, werden erfindungsgemäß bevorzugt.

Besonders vorteilhafte Wirkungen in Kombination mit den Ölkörpern zeigen erfindungsgemäße kosmetische Mittel, die als Isoparaffin eine Mischung aus Isoundecan, Isododecan und Isotridecan enthalten. In einer bevorzugten Ausführungsform sind erfindungsgemäße kosmetische Mittel daher dadurch gekennzeichnet, dass sie als Isoparaffin eine Mischung aus Isoundecan, Isododecan und Isotridecan, insbesondere eine Mischung aus Isododecan und Isotridecdan, enthalten.

Der Gewichtsanteil des Isoundecans, Isodedecans und Isotridecans am Gesamtgewicht aller in dem kosmetischen Mittel eingesetzten Isoparaffine beträgt vorzugsweise mehr als 62 Gew.-%, bevorzugt mehr als 72 Gew.-%, besonders bevorzugt mehr als 82 Gew.-% und insbesondere mehr als 92 Gew.-%. Besonders bevorzugt ist es, dass der Gewichtsanteil des Isodedecans und Isotridecans am Gesamtgewicht aller in dem kosmetischen Mittel eingesetzten Isoparaffine mehr als 60 Gew.-%, bevorzugt mehr als 70 Gew.-%, besonders bevorzugt mehr als 80 Gew.-% und insbesondere mehr als 90 Gew.-% beträgt.

Die Dichte bei 15°C (DIN 51 757 P.4; ASTM D 4052) bevorzugter Isoparaffingemische beträgt vorzugsweise 700 bis 820 kg/m³, bevorzugt 720 bis 800 kg/m³ und insbesondere 740 bis 780 kg/m³_{.}

Bevorzugte Isoparaffingemische zeichnen sich durch einen Siedebereich (DIN ES ISO 3405; ASTM D 86) zwischen 195°C und 250°C, bevorzugt zwischen 200°C und 245°C und insbesondere zwischen 205° und 240°C aus. Ein erfindungsgemäß besonders bevorzugtes Isoparaffingemisch ist kommerziell unter der Bezeichnung Pionier^{®} 2094 (H&R Group) erhältlich. Der Gewichtsanteil des Isoparaffins am Gesamtgewicht des kosmetischen Mittels beträgt vorzugsweise 10 bis 90 Gew.-%, bevorzugt 20 bis 80 Gew.-% und insbesondere 40 bis 60 Gew.-%.

Hinsichtlich ihrer kosmetischen Eigenschaften haben sich kosmetische Mittel als vorteilhaft erwiesen, bei denen der Gewichtsanteil von Esteröl und Isoparaffin am Gesamtgewicht des kosmetischen Mittels mindestens 20 Gew.-%, vorzugsweise mindestens 40 Gew.-%, bevorzugt zwischen 40 und 99,9, besonders bevorzugt zwischen 60 und 99,5 Gew.-% und insbesondere zwischen 80 und 99 Gew.-% beträgt.

Für die kosmetischen Eigenschaften der erfindungsgemäßen kosmetischen Mittel ist es weiterhin vorteilhaft, wenn das Gewichtsverhältnis von Esteröl zu Isoparaffin 6:1 bis 1:6, vorzugsweise 4:1 bis 1:4 und insbesondere 2:1 bis 1:2 beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 16 | Formel 17 | Formel 18 | Formel 19 | Formel 20 |
|---|---|---|---|---|---|
| Esteröl | 10 bis 90 | 20 bis 80 | 40 bis 60 | 20 | 50 |
| Isoparaffin * | 10 bis 90 | 20 bis 80 | 40 bis 60 | 78 | 47 |
| Misc | add 100 | add 100 | add 100 | add 100 | add 100 |

| | Formel 21 | Formel 22 | Formel 23 | Formel 24 | Formel 25 |
|---|---|---|---|---|---|
| 2-Ethylhexylpalmitat | 10 bis 90 | 20 bis 80 | 40 bis 60 | 20 | 50 |
| Isoparaffin * | 10 bis 90 | 20 bis 80 | 40 bis 60 | 78 | 47 |
| Misc | add 100 | add 100 | add 100 | add 100 | add 100 |

| | Formel 26 | Formel 27 | Formel 28 | Formel 29 | Formel 30 |
|---|---|---|---|---|---|
| 2-Ethylhexylpalmitat t | 10 bis 90 | 20 bis 80 | 40 bis 60 | 20 | 40 |
| Isopropylmyristat | 2 bis 35 | 5 bis 30 | 10 bis 25 | 20 | 20 |
| Isoparaffin *, Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. | | | | | |

Der zweite erfindungsgemäße Gegenstand enthält wie der erste Gegenstand ebenfalls ein Esteröl wie bereits zuvor beschrieben. Im Unterschied zum ersten Gegenstand hat sich jedoch gezeigt, dass die Menge an Esteröl erniedrigt werden kann. In diesem Falle haben sich demnach die folgenden Mengen als geeignet erwiesen:

Der Gewichtsanteil des Esteröls a) am Gesamtgewicht des kosmetischen Mittels beträgt vorzugsweise 0,1 bis 10 Gew.-%, vorzugsweise 0,2 bis 8,0 Gew.-% und insbesondere 0,5 bis 5,0 Gew.-%.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 31 | Formel 32 | Formel 33 | Formel 34 | Formel 35 |
|---|---|---|---|---|---|
| Esteröl | 0,1 bis 10 | 0,2 bis 8,0 | 0,5 bis 5,0 | 2,1 | 3,6 |
| Polymer *, Misc | add 100 | add 100 | add 100 | add 100 | add 100 |

| | Formel 36 | Formel 37 | Formel 38 | Formel 39 | Formel 40 |
|---|---|---|---|---|---|
| Ethylhexylpalmitat | 0,1 bis 10 | 0,2 bis 8,0 | 0,5 bis 5,0 | 0,5 | 2,5 |
| Polymer *, Misc | add 100 | add 100 | add 100 | add 100 | add 100 |

| | Formel 41 | Formel 42 | Formel 43 | Formel 44 | Formel 45 |
|---|---|---|---|---|---|
| 2-Ethylhexylpalmitat | 0,1 bis 5,0 | 0,1 bis 4,0 | 0,2 bis 3,0 | 0,5 | 2,5 |
| Isopropylmyristat | 0,1 bis 5,0 | 0,1 bis 4,0 | 0,2 bis 2,0 | 1,6 | 1,0 |
| Polymer *, Misc | add 100 | add 100 | add 100 | add 100 | add 100 |

| | | | | | |
|---|---|---|---|---|---|
| * polymere quartäre Ammoniumverbindung | | | | | |

Als zweiten wesentlichen Bestandteil des zweiten erfindungsgemäßen Gegenstandes der vorliegenden Erfindung enthalten die erfindungsgemäßen kosmetischen Mittel eine polymere quartäre Ammoniumverbindung. Die Gruppe der polymeren quartären Ammoniumverbindungen umfasst dabei erfindungsgemäß insbesondere die kationischen Polymere. Diese Polymere weisen erfindungsgemäß ein quartäres, also permanent kationisches Stickstoffatom mit vier Substituenten auf. Bei den polymeren quartären Ammoniumverbindungen handelt es sich mit anderen Worten um Polymere mit einer "permant" kationischen Ammoniumverbindung. Als "permanent kationisch" werden erfindungsgemäß solche Polymere bezeichnet, die unabhängig vom pH-Wert des Mittels eine kationische Gruppe aufweisen. Bevorzugte kationische Gruppen sind quartäre Ammoniumgruppen, die über eine C1-4-Kohlenwasserstoffgruppe an eine aus Acrylsäure, Methacrylsäure oder deren Derivaten aufgebaute Polymerhauptkette gebunden sind. Erfindungsgemäß bevorzugte Polymere zeichnen sich durch eine Ladungsdichte von mindestens 1 bis 7 meq/g aus. Eine Ladungsdichte von mindestens 2 bis 7 meq/g ist dabei bevorzugt. Besonders bevorzugt ist eine Ladungsdichte von mindestens gleich 3meq/g bis 7 meq/g. Bevorzugt sind solche Polymere, die eine ausreichende Löslichkeit in Wasser oder Alkohol besitzen, um in dem erfindungsgemäßen Mittel vollständig in Lösung zu gehen.

Die kationischen Polymere können Homo- oder Copolymere sein, wobei die quartären Stickstoffgruppen entweder in der Polymerkette oder vorzugsweise als Substituent an einem oder mehreren der Monomeren enthalten sind. Geeignete kationische Monomere sind ungesättigte, radikalisch polymerisierbare Verbindungen, welche mindestens eine kationische, quartäre Ammoniumgruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1-bis C7-Alkylgruppen, besonders bevorzugt C1- bis C3-Alkylgruppen sind.

Geeignete Polymere mit quartären Ammoniumgruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11). Weitere Beispiele für polymere quartäre Ammoniumverbindungen b) sind
- Copolymere des Vinylpyrrolidons mit quaternierten Derivaten des Dialkylaminoalkylacrylats und -methacrylats, beispielsweise Vinylpyrrolidon/Dimethylaminoethylmethacrylatmethosulfat Copolymere, wie sie unter den Handelsbezeichnungen Gafquat^{®} 755 N und Gafquat^{®} 734 von der Firma Gaf Co., USA vertrieben werden;
- Polyvinylpyrrolidon/Imidazoliminmethochlorid Copolymere, wie sie von der Firma BASF, Deutschland unter dem Handelsnamen Luviquat^{®} HM 550 vertrieben werden;
- Terpolymere aus Dimethyldiallylammoniumchlorid, Natriumacrylat und Acrylamid, das von der Firma Calgon/USA unter dem Handelsnamen Merquat^{®} Plus 3300 vertrieben wird;
- Vinylpyrrolidon/Methacrylamidopropyltrimethylammoniumchlorid Copolymer, das von der Firma ISP unter dem Handelsnamen Gafquat^{®} HS 100 vertrieben wird;
- polymere Dimethyldiallylammoniumsalze und deren Copolymere mit Estern und Amiden von Acrylsäure und Methacrylsäure, wie sie unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlich sind;
- Vinylpyrrolidon-Vinylimidazoliummethochlorid-Copolymere, wie sie unter den Bezeichnungen Luviquat^{®} FC 370, FC 550, FC 905 und HM 552 angeboten werden;
- die unter den Bezeichnungen Polyquaternium-24 (Handelsprodukt z. B. Quatrisoft^{®} LM 200), bekannten Polymere; sowie
- die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Besonders bevorzugte kosmetische Mittel sind dadurch gekennzeichnet, dass das se mindestens eine polymere quartäre Ammoniumverbindung ausgewählt aus der Gruppe der Homo- oder Copolymere des Methacryloyloxyethyltrimethylammoniumchlorids enthalten.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich.

Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Dieses Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCl-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Bevorzugte Copolymere des Methacryloyloxyethyltrimethylammoniumchlorids enthalten als nichtionogene Monomereinheiten Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich. Der Gewichtsanteil der polymeren quartären Ammoniumverbindung am Gesamtgewicht des kosmetischen Mittels beträgt vorzugsweise 0,1 bis 10 Gew.-%, bevorzugt 0,2 bis 8,0 Gew.-% und insbesondere 0,5 bis 5,0 Gew.-%.

Hinsichtlich ihrer pflegenden Eigenschaften haben sich erfindungsgemäße kosmetische Mittel als besonders vorteilhaft erwiesen, bei denen das Gewichtsverhältnis von Esteröl a) zu polymerer quartärer Ammoniumverbindung zwischen 8:1 und 1:8, vorzugsweise zwischen 6:1 und 1:6 und insbesondere zwischen 4:1 und 1:4 beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 46 | Formel 47 | Formel 48 | Formel 49 | Formel 50 |
|---|---|---|---|---|---|
| Esteröl | 0,1 bis 10 | 0,2 bis 8,0 | 0,5 bis 5,0 | 2,1 | 3,6 |
| Polymer * | 0,1 bis 10 | 0,2 bis 8,0 | 0,5 bis 5,0 | 1,3 | 2,0 |
| Misc | add 100 | add 100 | add 100 | add 100 | add 100 |

| | Formel 51 | Formel 52 | Formel 53 | Formel 54 | Formel 55 |
|---|---|---|---|---|---|
| Esteröl | 0,1 bis 10 | 0,2 bis 8,0 | 0,5 bis 5,0 | 2,1 | 3,6 |
| Polymer ** | 0,1 bis 10 | 0,2 bis 8,0 | 0,5 bis 5,0 | 1,3 | 2,0 |
| Misc | add 100 | add 100 | add 100 | add 100 | add 100 |

| | Formel 56 | Formel 57 | Formel 58 | Formel 59 | Formel 60 |
|---|---|---|---|---|---|
| 2-Ethylhexylpalmitat | 0,1 bis 10 | 0,2 bis 8,0 | 0,5 bis 5,0 | 0,5 | 2,5 |
| Polymer * | 0,1 bis 10 | 0,2 bis 8,0 | 0,5 bis 5,0 | 1,3 | 2,0 |
| Misc | add 100 | add 100 | add 100 | add 100 | add 100 |

| | Formel 61 | Formel 62 | Formel 63 | Formel 64 | Formel 65 |
|---|---|---|---|---|---|
| 2-Ethylhexylpalmitat | 0,1 bis 10 | 0,2 bis 8,0 | 0,5 bis 5,0 | 0,5 | 2,5 |
| Polymer ** | 0,1 bis 10 | 0,2 bis 8,0 | 0,5 bis 5,0 | 1,3 | 2,0 |
| Misc | add 100 | add 100 | add 100 | add 100 | add 100 |

| | Formel 66 | Formel 67 | Formel 68 | Formel 69 | Formel 70 |
|---|---|---|---|---|---|
| 2-Ethylhexylpalmitat | 0,1 bis 5,0 | 0,1 bis 4,0 | 0,2 bis 3,0 | 0,5 | 2,5 |
| Isopropylmyristat | 0,1 bis 5,0 | 0,1 bis 4,0 | 0,2 bis 2,0 | 1,6 | 1,0 |
| Polymer * | 0,1 bis 10 | 0,2 bis 8,0 | 0,5 bis 5,0 | 1,3 | 2,0 |
| Misc | add 100 | add 100 | add 100 | add 100 | add 100 |

| | Formel 71 | Formel 72 | Formel 73 | Formel 74 | Formel 75 |
|---|---|---|---|---|---|
| 2-Ethylhexylpalmitat | 0,1 bis 5,0 | 0,1 bis 4,0 | 0,2 bis 3,0 | 0,5 | 2,5 |
| Isopropylmyristat | 0,1 bis 5,0 | 0,1 bis 4,0 | 0,2 bis 2,0 | 1,6 | 1,0 |
| Polymer ** | 0,1 bis 10 | 0,2 bis 8,0 | 0,5 bis 5,0 | 1,3 | 2,0 |
| Misc | add 100 | add 100 | add 100 | add 100 | add 100 |

| | | | | | |
|---|---|---|---|---|---|
| *polymere quartäre Ammoniumverbindung **Poly(methacryloyloxyethyltrimethylammoniumchlorid) oder Acrylamid-Methacryloyloxyethyltrimethylammoniumchlorid-Copolymer | | | | | |

Der dritte Gegenstand dieser Anmeldung ist ein kosmetisches Mittel, enthaltend in einem kosmetisch akzeptablen Träger
a) mindestens ein Esteröl,
b) mindestens ein Isoparaffin aus der Gruppe Isodecan, Isoundecan, Isododecan, Isotridecan und Isotetradecan und
c) mindestens eine polymere quartäre Ammoniumverbindung.

Die zwingenden Bestandteile sind bereits zuvor beschrieben worden.

Bevorzugte kosmetische Mittel enthalten als weiteren Bestandteil einen Ölkörper. Zu den bevorzugten Ölkörpern zählen insbesondere:
- Die Silikonöle. Zur Gruppe der Silikonöle zählen insbesondere die Dimethicone, die aminofunktionellen Silikone sowie die Dimethiconole.

Die erfindungsgemäßen Dimethicone sind linear, bzw. linear verzweigt.

Lineare Dimethicone können durch die folgende Strukturformel (Si1) dargestellt werden:

(SiR¹₃) - O - (SiR²₂ - O - )ₓ - (SiR¹₃) (Si1)

Verzweigte Dimethicone können durch die Strukturformel (Si1.1) dargestellt werden:

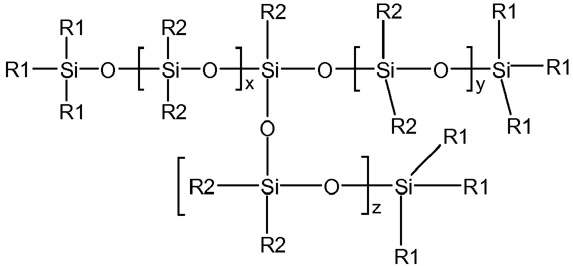

Bevorzugt als R¹ und R² sind Methyl, Phenyl und C2 bis C22 - Alkylreste, insbesondere jedoch Methyl. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen vorzugsweise zwischen 1000 D und 10000000 D. Die Viskositäten liegen vorzugsweise zwischen 100 und 10000000 cPs gemessen bei 25 °C mit Hilfe eines Glaskapillarviskosimeters nach der Dow Corning Corporate Testmethode CTM 0004 vom 20. Juli 1970. Besonders bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Dimethiconole (Si8) bilden eine weitere Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O - )ₓ - (SiOHR¹₂) (Si8 - I)

Verzweigte Dimethiconole können durch die Strukturformel (Si8 - II) dargestellt
werden:

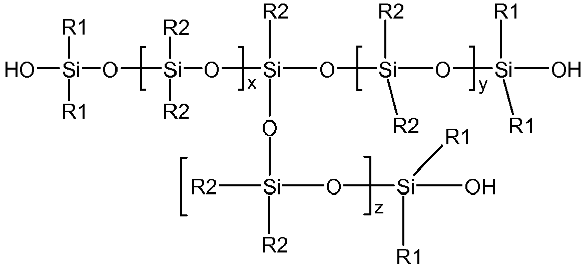

Bevorzugt als R¹ und R² sind Methyl, Phenyl und C2 bis C22 - Alkylreste, insbesondere Methyl. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethiconole liegen vorzugsweise zwischen 1000 D und 10000000 D. Die Viskositäten liegen vorzugsweise zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})ₓ(R_{c}SiO_{(4-c)/2})_{y}M (Si-2)

Beschrieben werden, wobei in der obigen Formel
R ein Kohlenwasserstoff oder ein Kohlenwasserstoffrest mit 1 bis etwa 6 Kohlenstoffatomen, vorzugsweise Methyl ist,
Q ein polarer Rest der allgemeinen Formel -R¹HZ, vorzugsweise ein Rest der Formel - CH₂CH₂CH₂NHCH₂CH₂NH ist, worin
R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
Z ein organischer, aminofunktioneller Rest ist, der mindestens eine aminofunktionelle Gruppe enthält, wobei es sich vorzugsweise um einen -NHCH₂CH ₂NH₂-Rest oder einen Rest der Formel -CH₂CH₂CH₂NHCH₂CH₂NH handelt;
a Werte im Bereich von etwa 0 bis etwa 2 annimmt,
b Werte im Bereich von etwa 1 bis etwa 3 annimmt,
a + b kleiner als oder gleich 3 ist, und
c eine Zahl im Bereich von etwa 1 bis etwa 3 ist, und
x eine Zahl im Bereich von 1 bis etwa 2.000, vorzugsweise von etwa 3 bis etwa 50 und am bevorzugtesten von etwa 3 bis etwa 25 ist, und
y eine Zahl im Bereich von etwa 20 bis etwa 10.000, vorzugsweise von etwa 125 bis etwa 10.000 und am bevorzugtesten von etwa 150 bis etwa 1.000 ist, und
M eine geeignete Silikon-Endgruppe ist, wie sie im Stande der Technik bekannt ist, vorzugsweise Trimethylsiloxy.

Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-, - OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Bevorzugte erfindungsgemäße kosmetische oder dermatologische Zubereitungen enthalten ein aminofunktionelles Silikon der Formel (Si-3)

R'ₐG₃₋ₐ-Si(OSiG₂)ₙ-(OSiG_{b}R'_{2-b})ₘ-O-SIG₃₋ₐR'ₐ (Si-3),

worin bedeutet:
G ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
a steht für eine Zahl zwischen 0 und 3, insbesondere 0;
b steht für eine Zahl zwischen 0 und 1, insbesondere 1,
m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
R' ist ein monovalenter Rest ausgewählt aus

   -Q-N(R")-CH₂-CH₂-N(R")₂

   -Q-N(R")₂

   -Q-N⁺(R")₃A⁻

   -Q-N⁺H(R")₂ A⁻

   -Q-N⁺H₂(R")A⁻

   -Q-N(R")-CH₂-CH₂-N⁺R"H₂A⁻,

   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat. Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), Dow Corning 939, Dow Corning 949, Dow Corning 959, DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric) sowie SLM-55067 (Hersteller: Wacker).

Besonders bevorzugte erfindungsgemäße Mittel sind dadurch gekennzeichnet, dass sie mindestens es ein aminofunktionelles Silikon der Formel (Si3-a) 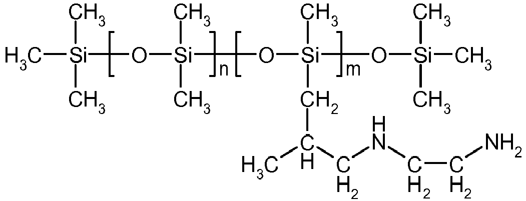 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 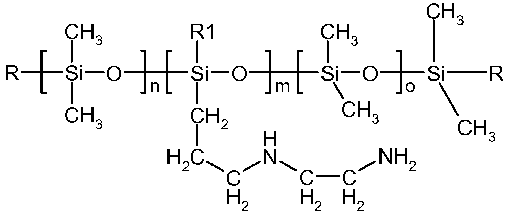 enthalten, worin
R für -OH, eine (gegebenenfalls ethoxylierte und/oder propoxylierte) (C₁ bis C₂₀)-Alkoxygruppe oder eine -CH₃-Gruppe steht,
R' für -OH, eine (C₁ bis C₂₀)-Alkoxygruppe oder eine -CH₃-Gruppe und
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), bezeichnet.

Ein besonders bevorzugtes funktionalisiertes Aminosilikon entspricht der folgenden Formel: 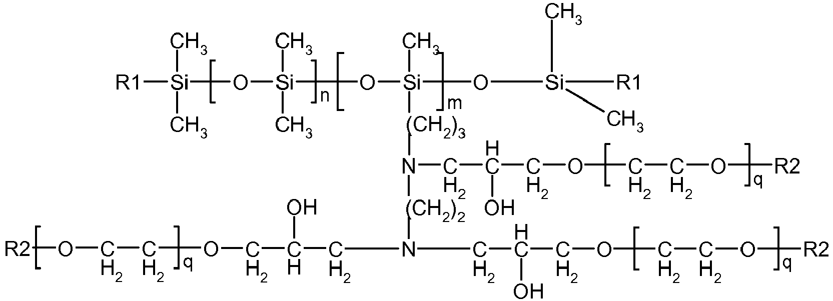
in welcher R1 steht für eine Methyl-, Ethyl-, Hydroxy-, Methoxy- oder Ethoxygruppe,
R2, steht für einen geradkettigen oder verzweigten C8 bis C24 Alkyl- oder Alkylenrest, vorzugsweise einen geradkettigen oder verzweigten C9 bis C22 Alkyl- oder Alkenylrest, besonders bevorzugt einen geradkettigen oder verzweigten C11 bis C18 Alkyl- oder Alkenylrest, höchst bevorzugt einen entsprechenden Alkylrest,
n und m jeweils für ganze Zahlen von 1 bis 1000 stehen und q steht jeweils für eine ganze Zahl von 2 bis 50, bevorzugt 4 bis 30, besonders bevorzugt 4 bis 18 und höchst bevorzugt von 4 bis 12.

Das Molekulargewicht derartiger Verbindungen beträgt 15.000 bis 2.000.000, gemessen mit einem Brookfield Rotationsviskosimeter RV, Spindel 5, bei 20 °C. Bevorzugt beträgt das Molgewicht 30.000 bis 1.750.000 und besonders bevorzugt 50.000 bis 1.500.000. Der Stickstoffgehalt der erfindungsgemäßen Silikone beträgt 0,03 bis 4,2 Gew.%, bevorzugt 0,1 bis 2,8 Gew.% und höchst bevorzugt 0,16 bis 1,4 Gew.%. Erfindungsgemäße aminofunktionelle kationische Silikone der obigen Formel können beispielsweise von der Fa. Clariant bezogen werden. Ein erfindungsgemäß höchst bevorzugtes Produkt ist unter der INCI-Bezeichnung Trideceth-9-Amodimethicone and Trideceth-12 im Handel erhältlich.

Eine erfindungsgemäß höchst bevorzugte Gruppe von aminofunktionalen Silikonen wird im Folgenden beschrieben. Ganz besonders bevorzugt wird daher als aminofunktionales Silikon mindestens ein 4-morpholinomethyl-substituiertes Silikon der Formel (V) verwendet, 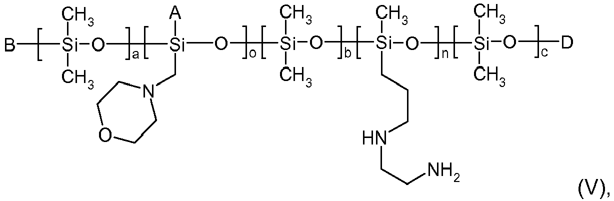 in der
A für eine über ein -O- gebundene Struktureinheit (I), (II) oder (III) 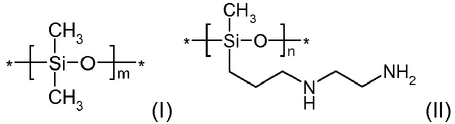 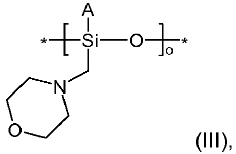 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.

Derartige aminofunktionale Silikone tragen die INCI - Bezeichnung Amodimethicone/Morpholinomethyl Silsesquioxane Copolymer. Ein besonders geeignetes Amodimethicone ist das Produkt mit der Handelsbezeichnung Wacker Belsil® ADM 8301E.

Die aminofunktionellen und wie zuvor beschriebenen Silikonpolymere sind in den erfindungsgemäßen Zusammensetzungen in Mengen von 0,01 bis 5 Gew.%, bevorzugt in Mengen von 0,05 bis 5 Gew.% und ganz besonders bevorzugt in Mengen von 0,1 bis 5 Gew.% enthalten. Die allerbesten Ergebnisse werden dabei mit Mengen von 0,1 bis 2,5 Gew.% jeweils bezogen auf die Gesamtzusammensetzung des jeweiligen Mittels erhalten.

Unabhängig davon, welche aminofunktionellen Silikone eingesetzt werden, sind erfindungsgemäße kosmetische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugt ist die Verwendung einer Mischung von Silikonölen. Diese Mischung von Silikonölen enthält vorzugsweise mindestens ein Dimethiconol. Besonders bevorzugt ist die Kombination eines Dimethiconols mit einem Dimethicon, vorzugsweise einem Cyclodimethicon. In bevorzugten Mischungen aus Dimethiconol und Cyclodimethicon beträgt das Gewichtsverhältnis von Dimethiconol zu Cyclodimethicon 1:2 bis 1:10, vorzugsweise 1:3 bis 1:9 und insbesondere 1:4 bis 1:8.

Bevorzugte erfindungsgemäße kosmetische Mittel sind dadurch gekennzeichnet, dass es sich bei dem Ölkörper um ein Silikonöl, vorzugsweise um ein Dimethiconol handelt. Der Gewichtsanteil des Silikonöls am Gesamtgewicht bevorzugter kosmetischer Mittel beträgt 15 bis 90 Gew.-%, vorzugsweise 20 bis 90 Gew.-%, besonders bevorzugt 25 bis 88 Gew.-% und insbesondere 30 bis 85 Gew.-%. Dies bedeutet, wenn nur ein Silikon verwendet wird, ist dieses mit mindestens 15 Gew.% bezogen auf die Gesamtzusammensetzung enthalten. Wenn mehrere erfindungsgemäßen Silikone verwendet werden, dann muss die Summe der Mengen der einzelnen Silikone mindestens 15 Gew.% betragen. Besonders bevorzugte kosmetische Mittel enthalten bezogen auf ihr Gesamtgewicht 2 bis 20 Gew.-%, vorzugsweise 3 bis 18 Gew.-%, besonders bevorzugt 4 bis 16 Gew.-% und insbesondere 5 bis 14 Gew.-% Dimethiconol.
- pflanzliche Öle. Beispiele für solche Öle sind Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Orangenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl, Weizenkeimöl, Wildrosenöl und die flüssigen Anteile des Kokosöls. Geeignet sind aber auch andere Triglyceridöle wie die flüssigen Anteile des Rindertalgs sowie synthetische Triglyceridöle. Bevorzugte erfindungsgemäße kosmetische Mittel sind dadurch gekennzeichnet, dass es sich bei dem Ölkörper c) um ein natürliches Öl handelt. Der Gewichtsanteil des natürlichen Öls am Gesamtgewicht bevorzugter kosmetischer Mittel beträgt 0,1 bis 10 Gew.-%, vorzugsweise 0,2 bis 8,0 Gew.% und insbesondere 0,4 bis 5,0 Gew.-%.
- flüssige Paraffinöle und synthetische Kohlenwasserstoffe sowie
- Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Fettalkohole. Zur Gruppe der bevorzugten Fettalkohole zählen die C₈ bis C₂₂ Fettalkohole, vorzugsweise die C₁₀ bis C₂₀ Fettalkohole und insbesondere die C₁₂ bis C₁₈ Fettalkohole. Bevorzugte erfindungsgemäße kosmetische Mittel sind dadurch gekennzeichnet, dass es sich bei dem Ölkörper c) um einen Fettalkohol handelt. Der Gewichtsanteil des Fettalkohols am Gesamtgewicht bevorzugter kosmetischer Mittel beträgt 0,1 bis 10 Gew.-%, vorzugsweise 0,2 bis 8,0 Gew.% und insbesondere 0,4 bis 5,0 Gew.-%.

Der der Gewichtsanteil des Ölkörpers am Gesamtgewicht erfindungsgemäß bevorzugter kosmetischer Mittel beträgt 30 bis 90 Gew.-%, vorzugsweise 35 bis 88 Gew.% und insbesondere 40 bis 85 Gew.-%.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 76 | Formel 77 | Formel 78 | Formel 79 | Formel 80 |
|---|---|---|---|---|---|
| Esteröl | 10 bis 80 | 20 bis 70 | 30 bis 60 | 20 | 50 |
| Isoparaffin * | 10 bis 80 | 20 bis 70 | 30 bis 60 | 78 | 47 |
| Cyclomethicon, Trioxolan | <5,0 | <2,0 | <0,5 | -- | -- |
| Misc | add 100 | add 100 | add 100 | add 100 | add 100 |

| | Formel 81 | Formel 82 | Formel 83 | Formel 84 | Formel 85 |
|---|---|---|---|---|---|
| Esteröl | 10 bis 80 | 20 bis 70 | 30 bis 60 | 20 | 50 |
| Isoparaffin ** | 10 bis 80 | 20 bis 70 | 30 bis 60 | 78 | 47 |
| Cyclomethicon, Trioxolan | <5,0 | <2,0 | <0,5 | -- | -- |
| Misc | add 100 | add 100 | add 100 | add 100 | add 100 |

| | Formel 86 | Formel 87 | Formel 88 | Formel 89 | Formel 90 |
|---|---|---|---|---|---|
| 2-Ethylhexylpalmitat | 10 bis 80 | 20 bis 70 | 30 bis 60 | 20 | 50 |
| Isoparaffin * | 10 bis 80 | 20 bis 70 | 30 bis 60 | 78 | 47 |
| Cyclomethicon, Trioxolan | <5,0 | <2,0 | <0,5 | -- | -- |
| Misc | add 100 | add 100 | add 100 | add 100 | add 100 |

| | Formel 91 | Formel 92 | Formel 93 | Formel 94 | Formel 95 |
|---|---|---|---|---|---|
| 2-Ethylhexylpalmitat | 10 bis 80 | 20 bis 70 | 30 bis 60 | 20 | 50 |
| Isoparaffin ** | 10 bis 80 | 20 bis 70 | 30 bis 60 | 78 | 47 |
| Cyclomethicon, Trioxolan | <5,0 | <2,0 | <0,5 | -- | -- |
| Misc | add 100 | add 100 | add 100 | add 100 | add 100 |

| | Formel 96 | Formel 97 | Formel 98 | Formel 99 | Formel 100 |
|---|---|---|---|---|---|
| 2-Ethylhexylpalmitat | 10 bis 90 | 20 bis 80 | 40 bis 60 | 20 | 40 |
| Isopropylmyristat | 2 bis 35 | 5 bis 30 | 10 bis 25 | 20 | 20 |
| Cyclomethicon, Trioxolan | <5,0 | <2,0 | <0,5 | -- | -- |
| Isoparaffin *, Misc | add 100 | add 100 | add 100 | add 100 | add 100 |

| | Formel 101 | Formel 102 | Formel 103 | Formel 104 | Formel 105 |
|---|---|---|---|---|---|
| 2-Ethylhexylpalmitat | 10 bis 90 | 20 bis 80 | 40 bis 60 | 20 | 40 |
| Isopropylmyristat | 2 bis 35 | 5 bis 30 | 10 bis 25 | 20 | 20 |
| Cyclomethicon, Trioxolan | <5,0 | <2,0 | <0,5 | -- | -- |
| Isoparaffin **, Misc | add 100 | add 100 | add 100 | add 100 | add 100 |

| | Formel 106 | Formel 107 | Formel 108 | Formel 109 | Formel 110 |
|---|---|---|---|---|---|
| Esteröl | 10 bis 80 | 20 bis 70 | 30 bis 60 | 20 | 50 |
| Isoparaffin * | 10 bis 80 | 20 bis 70 | 30 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Misc | add 100 | add 100 | add 100 | add 100 | add 100 |

| | Formel 111 | Formel 112 | Formel 113 | Formel 114 | Formel 115 |
|---|---|---|---|---|---|
| Esteröl | 10 bis 80 | 20 bis 70 | 30 bis 60 | 20 | 50 |
| Isoparaffin ** | 10 bis 80 | 20 bis 70 | 30 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Misc | add 100 | add 100 | add 100 | add 100 | add 100 |

| | Formel 116 | Formel 117 | Formel 118 | Formel 119 | Formel 120 |
|---|---|---|---|---|---|
| 2-Ethylhexylpalmitat | 10 bis 80 | 20 bis 70 | 30 bis 60 | 20 | 50 |
| Isoparaffin * | 10 bis 80 | 20 bis 70 | 30 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Misc | add 100 | add 100 | add 100 | add 100 | add 100 |

| | Formel 121 | Formel 122 | Formel 123 | Formel 124 | Formel 125 |
|---|---|---|---|---|---|
| 2-Ethylhexylpalmitat | 10 bis 80 | 20 bis 70 | 30 bis 60 | 20 | 50 |
| Isoparaffin ** | 10 bis 80 | 20 bis 70 | 30 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Misc | add 100 | add 100 | add 100 | add 100 | add 100 |

| | Formel 126 | Formel 127 | Formel 128 | Formel 129 | Formel 130 |
|---|---|---|---|---|---|
| 2-Ethylhexylpalmitat | 10 bis 80 | 20 bis 70 | 30 bis 60 | 20 | 40 |
| Isopropylmyristat | 10 bis 80 | 20 bis 70 | 30 bis 60 | 20 | 20 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Isoparaffin *, Misc | add 100 | add 100 | add 100 | add 100 | add 100 |

| | Formel 131 | Formel 132 | Formel 133 | Formel 134 | Formel 135 |
|---|---|---|---|---|---|
| 2-Ethylhexylpalmitat | 10 bis 80 | 20 bis 70 | 30 bis 60 | 20 | 40 |
| Isopropylmyristat | 10 bis 80 | 20 bis 70 | 30 bis 60 | 20 | 20 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Isoparaffin **, Misc | add 100 | add 100 | add 100 | add 100 | add 100 |

| | | | | | |
|---|---|---|---|---|---|
| *Isoparaffingemisch, umfassend mindestens ein Isoparaffin aus der Gruppe Isodecan, Isoundecan, Isododecan, Isotridecan und Isotetradecan. *Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. | | | | | |

Neben den zuvor beschriebenen Wirkstoffen können die erfindungsgemäßen kosmetischen Mittel weitere Inhaltsstoffe enthalten. Zur Gruppe dieser weiteren Inhaltsstoffe zählen insbesondere die kosmetisch wirksamen Wirk-, Hilfs- und Zusatzstoffe.

Als geeignete Wirk-, Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Die erfindungsgemäßen kosmetischen Mittel enthalten vorzugsweise einen kosmetischen Träger. Ein wässriger kosmetischer Träger enthält mindestens 50 Gew.-% Wasser. Unter wässrigalkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Besonders bevorzugt ist Wasser.

Besonders bevorzugte erfindungsgemäße kosmetische Mittel werden vorzugsweise als Wasser arme Anwendungsmischungen formuliert. Der Wassergehalt bevorzugter kosmetischer Mittel beträgt, bezogen auf ihr Gesamtgewicht, weniger als 10 Gew.-%, vorzugsweise weniger als 5,0 Gew.-%, besonders bevorzugt weniger als 1,0 Gew.-% und insbesondere weniger als 0,1 Gew.-%, wobei in ganz besonders bevorzugten kosmetischen Mitteln kein Wasser enthalten ist.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 136 | Formel 137 | Formel 138 | Formel 139 | Formel 140 |
|---|---|---|---|---|---|
| Esteröl | 10 bis 90 | 20 bis 80 | 40 bis 60 | 20 | 50 |
| Isoparaffin * | 10 bis 90 | 20 bis 80 | 40 bis 60 | 78 | 47 |
| Cyclomethicon, Trioxolan | <5,0 | <2,0 | <0,5 | -- | -- |
| Parfüm, Misc | add 100 | add 100 | add 100 | add 100 | add 100 |

| | Formel 141 | Formel 142 | Formel 143 | Formel 144 | Formel 145 |
|---|---|---|---|---|---|
| Esteröl | 10 bis 90 | 20 bis 80 | 40 bis 60 | 20 | 50 |
| Isoparaffin ** | 10 bis 90 | 20 bis 80 | 40 bis 60 | 78 | 47 |
| Cyclomethicon, Trioxolan | <5,0 | <2,0 | <0,5 | -- | -- |
| Parfüm, Misc | add 100 | add 100 | add 100 | add 100 | add 100 |

| | Formel 146 | Formel 147 | Formel 148 | Formel 149 | Formel 150 |
|---|---|---|---|---|---|
| 2-Ethylhexylpalmitat | 10 bis 90 | 20 bis 80 | 40 bis 60 | 20 | 50 |
| Isoparaffin * | 10 bis 90 | 20 bis 80 | 40 bis 60 | 78 | 47 |
| Cyclomethicon, Trioxolan | <5,0 | <2,0 | <0,5 | -- | -- |
| Parfüm, Misc | add 100 | add 100 | add 100 | add 100 | add 100 |

| | Formel 151 | Formel 152 | Formel 153 | Formel 154 | Formel 155 |
|---|---|---|---|---|---|
| 2-Ethylhexylpalmitat | 10 bis 90 | 20 bis 80 | 40 bis 60 | 20 | 50 |
| Isoparaffin ** | 10 bis 90 | 20 bis 80 | 40 bis 60 | 78 | 47 |
| Cyclomethicon, Trioxolan | <5,0 | <2,0 | <0,5 | -- | -- |
| Parfüm, Misc | add 100 | add 100 | add 100 | add 100 | add 100 |

| | Formel 156 | Formel 157 | Formel 158 | Formel 159 | Formel 160 |
|---|---|---|---|---|---|
| Isoparaffin * | 10 bis 90 | 20 bis 80 | 40 bis 60 | 20 | 40 |
| Isopropylmyristat | 2 bis 35 | 5 bis 30 | 10 bis 25 | 20 | 20 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | Formel 161 | Formel 162 | Formel 163 | Formel 164 | Formel 165 |
|---|---|---|---|---|---|
| Isoparaffin ** | 10 bis 90 | 20 bis 80 | 40 bis 60 | 20 | 40 |
| Isopropylmyristat | 2 bis 35 | 5 bis 30 | 10 bis 25 | 20 | 20 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | Formel 166 | Formel 167 | Formel 168 | Formel 169 | Formel 170 |
|---|---|---|---|---|---|
| Isoparaffin * | 10 bis 80 | 20 bis 70 | 40 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Esteröl, Parfüm | add 100 | add 100 | add 100 | add 100 | add 100 |

| | Formel 171 | Formel 172 | Formel 173 | Formel 174 | Formel 175 |
|---|---|---|---|---|---|
| Isoparaffin ** | 10 bis 80 | 20 bis 70 | 40 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| Esteröl, Parfüm | add 100 | add 100 | add 100 | add 100 | add 100 |

| | Formel 176 | Formel 177 | Formel 178 | Formel 179 | Formel 180 |
|---|---|---|---|---|---|
| Isoparaffin * | 10 bis 80 | 20 bis 70 | 40 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | Formel 181 | Formel 182 | Formel 183 | Formel 184 | Formel 185 |
|---|---|---|---|---|---|
| Isoparaffin ** | 10 bis 80 | 20 bis 70 | 40 bis 60 | 78 | 47 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | Formel 186 | Formel 187 | Formel 188 | Formel 189 | Formel 190 |
|---|---|---|---|---|---|
| Isoparaffin * | 10 bis 80 | 20 bis 70 | 30 bis 60 | 20 | 40 |
| Isopropylmyristat | 10 bis 80 | 20 bis 70 | 30 bis 60 | 20 | 20 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | Formel 191 | Formel 192 | Formel 193 | Formel 194 | Formel 195 |
|---|---|---|---|---|---|
| Isoparaffin ** | 10 bis 80 | 20 bis 70 | 30 bis 60 | 20 | 40 |
| Isopropylmyristat | 10 bis 80 | 20 bis 70 | 30 bis 60 | 20 | 20 |
| Natürliches Öl | 0,1 bis 10 | 0,2 bis 8,0 | 0,4 bis 5,0 | 0,2 | 0,6 |
| Cyclomethicon, Trioxolan | <5,0 | <2,0 | <0,5 | -- | -- |
| 2-Ethylhexylpalmitat, Parfüm | add 100 | add 100 | add 100 | add 100 | add 100 |

| | | | | | |
|---|---|---|---|---|---|
| * Isoparaffingemisch, umfassend mindestens ein Isoparaffin aus der Gruppe Isodecan, Isoundecan, Isododecan, Isotridecan und Isotetradecan. ** Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. | | | | | |

Wie eingangs ausgeführt, eignet sich die erfindungsgemäße Wirkstoffkombination aus Isoparaffin und Esteröl und gegebenenfalls einer polymeren quarternären Verbindung insbesondere als Triloxanersatz. Bevorzugte erfindungsgemäße kosmetische Mittel sind daher dadurch gekennzeichnet, dass sie bezogen auf ihr Gesamtgewicht, weniger als 5,0 Gew.-%, vorzugsweise weniger als 2,0 Gew.-%, bevorzugt weniger als 0,5 Gew.-% und insbesondere kein Trisiloxan enthalten.

Wie eingangs ausgeführt, eignen sich die erfindungsgemäßen Mittel insbesondere zur Haarpflege. Ein weiterer Gegenstand der vorliegenden Anmeldung ist daher ein Verfahren zur Behandlung keratinischer Fasern dadurch gekennzeichnet, dass ein erfindungsgemäßes kosmetisches Mittel auf die getrockneten und/oder Handtuchfeuchten keratinischen Fasern aufgetragen wird.

Das erfindungsgemäße Haarbehandlungsmittel wird bevorzugt unmittelbar vor, während oder nach einer oxidativen oder tensidischen Haarbehandlung verwendet. Als unmittelbar vor der oxidativen oder tensidischen Haarbehandlung versteht sich im Sinne der Erfindung eine Anwendung, an die sich direkt die oxidative oder tensidische Haarbehandlung anschließt, wobei das erfindungsgemäße Haarbehandlungsmittel zuvor vom Haar gespült oder bevorzugt auf dem Haar belassen wurde und das Haar bevorzugt noch nass ist.

Als nach der oxidative oder tensidischen Haarbehandlung versteht sich im Sinne der Erfindung eine Anwendung, welche sich entweder direkt an die oxidative oder tensidische Haarbehandlung anschließt, wobei das erfindungsgemäße Haarbehandlungsmittel nach dem Abspülen des oxidativ oder tensidisch wirkenden Mittels auf das bevorzugt noch nasse, handtuchtrockene Haar aufgetragen wird, oder erst nach mehreren Stunden oder Tagen auf das trockene oder nasse Haar aufgetragen wird. In beiden Fällen kann das erfindungsgemäße Haarbehandlungsmittel hierbei nach einer Einwirkzeit von wenigen Sekunden bis hin zu 45 Minuten wieder ausgespült werden oder vollständig auf dem Haar verbleiben.

Die Wirkung des erfindungsgemäßen Haarbehandlungsmittels entfaltet sich sogar während der oxidativen oder tensidischen Haarbehandlung und hält überraschenderweise auch nach intensivem Auswaschen des erfindungsgemäßen Haarbehandlungsmittels an.

Ein weiterer Gegentand der vorliegenden Anmeldung ist die Verwendung eines erfindungsgemäßen kosmetischen Mittels zur Pflege keratinischer Fasern. Beansprucht wird dabei insbesondere die Verwendung eines erfindungsgemäßen kosmetischen Mittels
- zur Verbesserung der Nass- und Trockenkämmbarkeiten keratinischer Fasern,
- zur Verbesserung des Glanzes keratinischer Fasern,
- zur Verbesserung des Feuchtehaushaltes keratinischer Fasern,
- zum Schutz der keratinischen Fasern vor oxidativen Schädigungen, insbesondere im Zuge oxidativer Haarbehandlungen,
- zum Verhindern des Nachfettens keratinischer Fasern,
- zur Erhöhung der Waschbeständigkeit gefärbter keratinischer Fasern,
- zur Erzielung eines erhöhten Frisurenhaltes über mindestens 48 h, insbesondere 24 h hinweg,
- zur Verbesserung der sogenannten curl-retention keratinischer Fasern,
- zur Verbesserung des Griffs keratinischer Fasern,
- zur Verbesserung der Regeneration keratinischer Fasern.

Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Messparameter können durch den Fachmann sensorisch beurteilt oder durch Messeinrichtungen quantifiziert werden.

Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Als Griff definiert sich die Taktilität eines Faserkollektivs, wobei der Fachmann sensorisch die Parameter Fülle und Geschmeidigkeit des Kollektivs fühlt und bewertet.

### Beispiele

Alle Mengenangaben sind, soweit nicht anders vermerkt, Gewichtsteile. Die folgenden Rezepturen wurden unter Anwendung bekannter Herstellungsverfahren bereitgestellt

| **Haaröle** | Öl 1 | Öl 2 | Öl 3 | Öl 4 |
|---|---|---|---|---|
| Cyclomethicone and Dimethicone | 35,0 | 50,0 | 80,0 | ---- |
| C10-C13 Isoparaffin | 5,0 | 10,0 | 15,0 | 50,0 |
| Isopropylmyristat | 20,0 | ---- | ---- | ---- |
| Macadamianussöl | 0,2 | 0,1 | 0,1 | 0,1 |
| Marulaöl | ---- | 0,1 | 0,1 | 0,1 |
| Arganöl | ---- | 0,1 | 0,1 | 0,1 |
| Aprikosenkernöl | ---- | 0,1 | 0,1 | 0,1 |
| Mandelöl | ---- | 0,1 | 0,1 | 0,1 |
| Olivenöl | ---- | 0,1 | 0,1 | 0,1 |
| Sesamöl | ---- | 0,1 | 0,1 | 0,1 |
| Ethylhexylpalmitat | Ad 100 | Ad 100 | Ad 100 | Ad 100 |
| Parfüm. Konservierung, Wasser, pH-Wert Einstellung | q.s. | q.s | q.s. | q.s. |

| **Haarkuren** | Kur 1 | Kur 2 | Kur 3 | |
|---|---|---|---|---|
| Isopropylmyristat | 1,0 | 1,6 | 1,6 | |
| Glycerinmonostearat | 0,5 | 0,7 | ---- | |
| Quaternium-87 | 2,0 | 1,0 | 1,0 | |
| Distearoylethyl Hydroxyethylmonium Methosulfate | 1,0 | 0,75 | 0,75 | |
| Cetearylalcohol | 4,8 | 0,25 | 3,8 | |
| Ceteareth-20 | 0,5 | ---- | 1,0 | |
| Stearamidopropyldimethylamine | 0,8 | 0,7 | 0,7 | |
| Polyquaternium-37 | 1,3 | 2,0 | 0,3 | |
| Dimethicone and Dimethiconol | ---- | 0,5 | ---- | |
| Ethylhexalpalmitate | 2,5 | 1,5 | 1,5 | |
| C10-C131soparaffin | 0,5 | 0,5 | 1,5 | |
| Glycerin | 0,8 | 1,0 | 1,0 | |
| Aprikosenkernöl | ---- | 0,5 | 3,0 | |
| Proteinhydrolysat | 0,15 | ---- | 0,2 | |
| Panthenol | 0,2 | 0,3 | 0,3 | |
| Parfüm. Puffer, Konservierung, Wasser | Ad 100 | Ad 100 | Ad 100 | |

## Patentansprüche

1. Kosmetisches Mittel, enthaltend in einem kosmetisch akzeptablen Träger
a) mindestens ein Esteröl
b) mindestens ein Isoparaffin aus der Gruppe Isodecan, Isoundecan, Isododecan, Isotridecan und Isotetradecan.

2. Kosmetisches Mittel, enthaltend in einem kosmetisch akzeptablen Träger
a) mindestens ein Esteröl
b) mindestens eine polymere quartäre Ammoniumverbindung.

3. Kosmetisches Mittel, enthaltend in einem kosmetisch akzeptablen Träger
a) mindestens ein Esteröl,
b) mindestens ein Isoparaffin aus der Gruppe Isodecan, Isoundecan, Isododecan, Isotridecan und Isotetradecan und
c) mindestens eine polymere quartäre Ammoniumverbindung.

4. Kosmetisches Mittel nach einem der Ansprüche 2 oder 3, **dadurch gekennzeichnet, dass** das kosmetische Mittel mindestens eine polymere quartäre Ammoniumverbindung ausgewählt aus der Gruppe der Polymere des Methacryloyloxyethyltrimethylammoniumchlorids enthält.

5. Kosmetisches Mittel nach einem der Ansprüche 2 bis 4, **dadurch gekennzeichnet, dass** der Gewichtsanteil der polymeren quartären Ammoniumverbindung am Gesamtgewicht des kosmetischen Mittels 0,1 bis 10 Gew.-%, vorzugsweise 0,2 bis 8,0 Gew.-% und insbesondere 0,5 bis 5,0 Gew.-% beträgt.

6. Kosmetisches Mittel nach einem der Ansprüche 1 bis 5, **dadurch gekennzeichnet, dass** als Esteröl 2-Ethylhexylpalmitat eingesetzt wird.

7. Kosmetisches Mittel nach einem der Ansprüche 1 oder 3 bis 6, **dadurch gekennzeichnet, dass** das kosmetische Mittel mindestens ein Isoparaffin ausgewählt aus der Gruppe Isoundecan, Isododecan, Isotridecan enthält.

8. Kosmetisches Mittel nach einem der vorgehenden Ansprüche, **dadurch gekennzeichnet, dass** das Gewichtsverhältnis von Esteröl zu Isoparaffin 6:1 bis 1:6, vorzugsweise 4:1 bis 1:4 und insbesondere 2:1 bis 1:2 beträgt.

9. Kosmetisches Mittel nach einem der vorgehenden Ansprüche, **dadurch gekennzeichnet, dass** der Wassergehalt des kosmetischen Mittels, bezogen auf sein Gesamtgewicht, weniger als 10 Gew.-%, vorzugsweise weniger als 5,0 Gew.-%, besonders bevorzugt weniger als 1,0 Gew.-% und insbesondere weniger als 0,1 Gew.-% beträgt.

10. Kosmetisches Mittel nach einem der vorgehenden Ansprüche, **dadurch gekennzeichnet, dass** das kosmetische Mittel als weiteren Bestandteil mindestens einen weiteren Ölkörper enthält.

11. Verwendung eines kosmetischen Mittels nach einem der Ansprüche 1 bis 10 zur Pflege keratinischer Fasern.
